# EUROPÄISCHE PATENTSCHRIFT

(11) **EP 0 705 845 B1**
(45) Veröffentlichungstag und Bekanntmachung des Hinweises auf die Patenterteilung: **02.05.2003**
(21) Anmeldenummer: 95115650.4
(22) Anmeldetag: 04.10.1995
(51) Int. Cl.: C07K 14/525, C07K 1/36, C08B 37/00

(54) **Verfahren zur simultanen Entfernung von Tumor-Nekrose-Faktor alpha und bakteriellen Lipopolysacchariden aus einer wässrigen Flüssigkeit**
Process of simultaneous removal of tumor necrosis factor and bacterial lipopolysaccharides from an aqueous solution
Procédé de d'élimination simultanée du facteur de nécrose tumorale et de lipopolysaccharides bactériens à partir d'une solution aqueuse

(30) Priorität: 05.10.1994 DE 4435612
(43) Veröffentlichungstag der Anmeldung: 10.04.1996
(73) Patentinhaber: B. BRAUN MELSUNGEN AG, 34212 Melsungen (DE)
(72) Erfinder: Boos, Karl-Siegfried, Prof. Dr., D-82131 Gauting (DE); Seidel, Dietrich, Prof. Dr. med., D-82340 Feldafing (DE); Trautwein, Annette, D-67454 Hassloch (DE); Morsch, Gerold, D-34628 Willinghausen (DE)
(74) Vertreter: Böhm, Brigitte, Dipl.-Chem. Dr.

(56) Entgegenhaltungen:
- EP-A- 0 424 698
- EP-A- 0 592 989
- DE-A- 3 135 814

## Beschreibung

Die vorliegende Erfindung betrifft ein Verfahren zur Entfernung von Tumor-Nekrose-Faktor α oder/und LPS aus einer wäßrigen Flüssigkeit, vorzugsweise Körperflüssigkeit und insbesondere Blut, Blutplasma oder Blutserum in einem extrakorporalen Perfusionssystem.

Die selektive Eliminierung des Tumor-Nekrose-Faktors α (TNFα) und/oder von bakteriellen Lipopolysacchariden (LPS, Synonym: Endotoxine) aus Humanblut ist aus medizinischer Sicht insbesondere für die Behandlung einer schweren Sepsis mit gestörter Organperfusion, mit Hypotension oder mit Multi-organversagen erwünscht (Intensivtherapie bei Sepsis und Multiorganversagen, Schuster, H.-P., ed., 1993, Springer Verlag, Berlin). Die Prognose des septischen Schocks ist unter der derzeitigen Standardtherapie schlecht. Bei bis zu 50 % der Patienten muß trotz aller therapeutischen Bemühungen mit einem letalen Ausgang gerechnet werden. In den USA wird die Anzahl der durch einen septischen Schock hervorgerufenen Todesfälle auf ca. 100 000 pro Jahr geschätzt (Parillo, J.E., "Septic Shock in Humans" in: Annals of Internal Medicine, Vol. 113, No. 3, 1990, 227-242).

Die septischen Komplikationen entstehen nach einer Infektion des Menschen durch gramnegative Bakterien. Die Invasion der Bakterien in die Blutbahn führt beim Zerfall der Bakterien zur Freisetzung von LPS aus der äußeren Bakterienzellwand. Die bakteriellen Lipopolysaccharide besitzen eine stäbchenartige Form und sind aus drei strukturell unterschiedlichen Regionen aufgebaut. Der Träger der toxischen Eigenschaften ist das Lipid-A. Diese Subregion mit einem Molekulargewicht von 2000 Dalton besteht aus einem phosphorylierten D-Glucosamin-Disaccharid, an das ester- und amidartig mehrere langkettige Fettsäuren gebunden sind (Bacterial Endotoxic Lipopolysaccharides, Morrison, D.C., Ryan, J.L. eds., 1992, CRC Press).

Die bakteriellen Lipopolysaccharide (LPS) sind als initiierende Mediatoren die wichtigsten Toxine in der Pathogenese des septischen Schocks. Das klinische Bild der Sepsis korreliert in den meisten Fällen mit dem Verlauf und der Höhe der LPS-Konzentration im Blut der Patienten (Nitsche, D. et al., Intensive Care Med., 12 Suppl., 1986, 185 ff.). Die Lipopolysaccharide stimulieren die als Makrophagen bezeichneten Freßzellen im Organismus zur Produktion und Freisetzung des TNFα.

Der TNFα ist ein Hormon des Immunsystems und zählt zur Klasse der Cytokine. Die biologisch wirksame Form des TNFα besteht aus einem Aggregat dreier identischer Polypeptidketten (157 Aminosäuren, Molekulargewicht: 17,4 x 10³ Dalton) (Ziegler, E.J., N. Engl. J. Med. 318, 1988, 1533 ff.). TNFα nimmt unter den Cytokinen eine Schlüsselrolle hinsichtlich der Pathogenese des septischen Schocks ein.

So besteht bei Patienten mit beispielsweise einer Meningokokken-Sepsis eine Korrelation zwischen der TNF-Konzentration im Plasma und dem Grad der septischen Schock-Symptome bzw. dem späteren Todeseintritt (Grau, G.E. et al., Immunol. Rev. 112, 1989, 49 ff.). Erhöhte TNF-Plasmakonzentrationen finden sich auch bei Patienten mit parasitären Erkrankungen und anderen Infektionen (Scuderi, P. et al., Lancet II, 1986, 1229 ff.).

Vom intensiv-therapeutischen Standpunkt ist daher eine selektive Elimination der pathogenen Blutbestandteile LPS und TNFα wünschenswert. Dies gilt insbesondere auch deshalb, weil beispielsweise die Gabe von hochwirksamen Antibiotika (Shenep, J.L., Morgan, K.A., J. Infect. Dis. 150, 1984, 380 ff.), von Immunglobulinen (Schedel, F. et al., Crit. Care Med. 19, 1991, 1104 ff.) oder von monoklonalen Antikörpern gegen LPS und TNFα (Werdan, K., Intensivmed. 30, 1993) 201 ff.) die Prognose (Überlebensrate) nicht wesentlich verbessern konnte.

Bislang wurden für die Elimination von Lipopolysacchariden (Endotoxinen) aus biologischen Flüssigkeiten poröse Adsorbermaterialien, wie beispielsweise chemisch modifizierte Polymere (US 3,959,128, US 4,491,660, EP 0 362 876), Aktivkohle und Derivate (DE 32 30 540 A1), Polyethylenimin-beschichtete Perlcellulose (DE-OS 41 13 602 A1) und poröse Trägermaterialien mit immobilisiertem Polymyxin B (US 4,661,260, US 4,576,928) beschrieben. Diese Materialien eignen sich jedoch nicht für die Verwendung in einem extrakorporalen Perfusionssystem, da neben LPS auch nicht-pathogene oder protektive Plasmakomponenten entfernt werden. Die mit dem cyclischen Decapeptid Polymyxin B modifizierten Materialien weisen einerseits die erwünschte Selektivität auf, andererseits ist deren klinische Anwendung sehr problematisch, da der Ligand eine sehr nephrotoxische und neurotoxische Substanz ist (Barkow, D. in: Intensivtherapie bei Sepsis und Multiorganversagen, Schuster, H.-P., ed., 1993, 132 ff., Springer-Verlag, Berlin).

Für die Gewinnung und/oder Elimination des TNFα aus biologischen Flüssigkeiten wurden bislang nur poröse Adsorbermaterialien mit Kationenaustauscher-Eigenschaften beschrieben (Bak, S.J. et al., Korea 9209520, Boos, K.-S. et al., DE 43 31 358 A1).

Alle bisher für eine extrakorporale Sepsis-Therapie potentiell verfügbaren Möglichkeiten weisen den entscheidenden Nachteil auf, der darin liegt, daß nur ein einzelnes Pathogen eliminiert wird.

Um den klinischen Verlauf des septischen Krankheitsbildes günstig zu beeinflussen, ist es jedoch aus pathophysiologischer und therapeutischer Sicht wünschenswert, beide pathogenen Blutbestandteile (LPS und TNFα) simultan aus dem Kreislauf des Patienten zu entfernen.

Durch diese Maßnahme wird die biologische Mediatorkaskade initial unterbrochen und die fatalen synergistischen Effekte der beiden Pathogene TNFα und LPS wirkungsvoll ausgeschaltet.

Die Aufgabe der vorliegenden Erfindung war es daher, ein effektives Verfahren bereitzustellen, um extrakorporal beide Faktoren LPS und TNFα simultan aus Blut, Serum oder Plasma zu entfernen.

Gelöst wird diese Aufgabe durch ein Verfahren zur Entfernung von Tumor-Nekrose-Faktor α (TNFα) oder/und bakteriellen Lipopolysacchariden (LPS) aus einer Körperflüssigkeit, insbesondere Blut, Blutplasma oder Serum, in einem extrakorporalen Perfusionssystem, welches dadurch gekennzeichnet ist, daß man nach gegebenenfalls nötiger Entfernung von korpuskulären Blutbestandteilen
(a) den pH-Wert der Körperflüssigkeit auf pH < 6 einstellt,
(b) ein Fällungsreagenz in Form eines Polyanions zugibt,
(c) gefällte Substanzen abfiltriert oder/und abzentrifugiert, und
(d) die resultierende Flüssigkeit über einen Anionenaustauscher leitet.

Das erfindungsgemäße Verfahren ist weitgehend analog zu dem in EP 0 166 324, DE 33 10 727, EP 0 074 610, EP 0 180 720 und US 4,935,204 beschriebenen klinischen Aphereseverfahren (Heparininduzierte extrakorporale LDL-Präzipitation; HELP), mit dessen Hilfe die Blutbestandteile Low Density Lipoproteine (LDL) und Fibrinogen aus dem Plasma von Patienten selektiv entfernt werden können.

Das HELP-Verfahren wird bisher zur chronischen Behandlung von Patienten mit einer angeborenen Fettstoffwechselstörung, der schweren primären Hypercholesterinämie, eingesetzt. Diese Patienten haben ein außerordentlich hohes Risiko, an koronarer Herzerkrankung frühzeitig zu versterben. Mit dem HELP-Verfahren wurden bereits über 50.000 Einzelbehandlungen an etwa 260 Patienten durchgeführt (Seidel, D., The HELP-Report 1994, MMV Medizin Verlag, München (1994)).

Bei dem HELP-Verfahren wird über eine Membran abfiltriertes Plasma des Patienten zunächst im Verhältnis 1:1 mit einem 0,2 M Acetat-Essigsäure-Puffer (pH 4,85) in Gegenwart von Heparin (100 IU/ml) versetzt. Das unter diesen Bedingungen bei pH 5,12 erzeugte Präzipitat aus LDL, Heparin und Fibrinogen wird unter rezirkulierenden Bedingungen auf einer Polycarbonat-Filterkerze quantitativ abgetrennt. In einem weiteren Schritt wird das überschüssige Fällungsreagenz Heparin an einem DEAE-Cellulose-Anionenaustauscher selektiv adsorbiert und damit entfernt. Die Plasma-Puffer-Mischung wird anschließend einer Bicarbonat-Dialyse und Ultrafiltration unterzogen, um den pH-Wert, das Volumen und den Acetat-Gehalt auf physiologische Verhältnisse einzustellen. Das behandelte Plasma wird anschließend mit den Blutzellen vermischt und dem Patienten zurückgegeben. Mit dem HELP-Verfahren können derzeit bis zu 3 Liter Plasma behandelt werden. LDL und Fibrinogen werden quantitativ aus dem behandelten Plasma entfernt.

Überraschenderweise wurde im Rahmen der vorliegenden Erfindung festgestellt, daß bei extrakorporaler Behandlung von Blut, Serum oder Plasma von Patienten, welche das klinische Bild einer Sepsis bis hin zum septischen Schock zeigen, mit analogen Schritten wie beim HELP-Verfahren eine effiziente Entfernung beider pathogener Blutbestandteile, LPS und TNFα, simultan möglich ist. Hierdurch wird erstmals eine erfolgsversprechende klinische Sepsistherapie ermöglicht, die aufgrund der hohen Anzahl von Todesfällen bei diesem Krankheitsbild dringend benötigt wird.

Bei dem erfindungsgemäßen Verfahren wird durch Zugabe eines organischen oder anorganischen Polyanions und insbesondere von Heparin eine sehr effektive Fällung von TNFα erreicht, welches dann abfiltriert oder abzentrifugiert wird. In einem zweiten Schritt wird überschüssiges Polyanion durch Bindung an einen Anionenaustauscher entfernt, wobei im Rahmen der vorliegenden Erfindung überraschenderweise sehr effektiv gleichzeitig LPS entfernt wird. Durch Anwendung des erfindungsgemäßen Verfahrens können daher beide Sepsis-verursachende oder -begleitende Substanzen sehr effektiv aus dem Blut eliminiert werden.

Im Rahmen der vorliegenden Erfindung ist es bevorzugt, den pH-Wert der Körperflüssigkeit auf einen Wert zwischen 4,0 und 5,8 und insbesondere besonders bevorzugt auf 5,05 bis 5,25 einzustellen. Dies erfolgt zweckmäßigerweise durch Zugabe eines Puffers, insbesondere eines Citratpuffers, eines Lactatpuffers, eines Acetatpuffers oder von Gemischen hiervon. Die Verdünnung des Plasmas durch derartige eingesetzte Puffer liegt vorzugsweise bei einem Verhältnis von Körperflüssigkeit zu Pufferlösung von 1:5 bis 5:1.

Das erfindungsgemäß verwendete Polyanion kann aus der Gruppe der Substanzen Heparin, hydrolysiertes Heparin, sulfatiertes Glucosaminoglycan oder sulfatierte Polysaccaride oder Mischungen dieser Substanzen ausgewählt werden. Besonders bevorzugt wird als Polyanion Heparin verwendet.

Das Polyanion wird in einer solchen Menge eingesetzt, daß das in der Körperflüssigkeit vorhandene TNFα möglichst quantitativ ausgefällt wird. Dies ist vorzugsweise eine Menge von 0,001 bis 10 mg/ml Körperflüssigkeit und für Heparin und seine Derivate insbesondere eine Menge von 10 bis 400 IU/ml Körperflüssigkeit.

Die gefällten Substanzen werden dann abfiltriert oder abzentrifugiert. Hierzu können alle für extrakorporale Perfusionssysteme geeignete Filter oder Durchflußzentrifugen zur Anwendung kommen. Besonders bevorzugt sind Filterkerzen, welche eine besonders effiziente Abtrennung der ausgefällten Substanzen gewährleisten. Besonders bevorzugt werden hierbei Filtermaterialien verwendet, welche eine mittlere Porengröße von 0,01 bis 1,0 µm aufweisen.

Die selektive Abtrennung von überschüssigem Polyanion und erfindungsgemäß LPS erfolgt über einen Anionenaustauscher.

Vorzugsweise wird im Rahmen der Erfindung ein Anionenaustauscher verwendet, der ein Grundträgermaterial aufweist, welches aus porösem Glas oder/und mit organischen Polymerisaten oder Copolymerisaten beschichtetem Kieselgel, quervernetzten Kohlehydraten oder/und organischen Polymerisaten oder Copolymerisaten besteht.

Als Anionenaustauscher wird ferner vorzugsweise ein Material verwendet, das als funktionelle Gruppen Kationen oder natürliche, synthetische oder halbsyntetische Polykationenketten enthält, wobei Polykationenketten in linearer oder verzweigter Form vorliegen können. Besonders bevorzugt verwendet man als Kationen bzw. Polykationenketten tertiäre oder/und quartäre Amine. Besonders bevorzugte Anionenaustauscher sind quervernetzte oder/und mikrogranuläre Dialkylaminoalkyl-, Dialkylaminoaryl-, Trialkylammoniumalkyl- oder Trialkylammoniumaryl-Cellulosen oder/und Dialkylaminoalkyl-, Dialkylaminoaryl-, Trialkylammoniumalkyl- oder Trialkylammoniumaryl-modifizierte organische Polymere oder Copolymere. Besonderes bevorzugte Verbindungen sind hierbei DEAE-Cellulosen und EMD-Fraktogele.

Nach der letztendlichen Entfernung der Substanzen TNFα und LPS wird in einer bevorzugten Ausführungsform der Erfindung durch Ultrafiltration der ursprüngliche Wassergehalt der Flüssigkeit wieder hergestellt und/oder durch einen zusätzlichen Dialyseschritt und/oder durch Zugabe eines geeigneten Puffers, wie beispielsweise Bicarbonat-Puffer, der physiologische pH-Wert regeneriert.

Bei der Einstellung des pH-Werts durch eine Pufferlösung wird nämlich das Blut bzw. Blutplasma so weit verdünnt, daß es nicht ohne weiteres an den Patienten zurückgegeben werden könnte. Bevorzugt wird daher eine Dialyse durchgeführt, und insbesondere wird diese Dialyse in Schritt (e) gegen einen Bicarbonatpuffer durchgeführt. Auch der Wassergehalt der Flüssigkeit muß wieder hergestellt werden, bevor das Blut oder das Blutplasma an den Patienten zurückgegeben werden kann. Daher ist es besonders bevorzugt, eine Ultrafiltration durchzuführen, wobei jedoch auch jede andere Möglichkeit zur Regenerierung des ursprünglichen Wassergehalts der Körperflüssigkeit geeignet erscheint.

Im Zusammenhang mit einer späteren Rückführung des von TNFα und LPS gereingten Plasmas an Patienten ist es für den Fachmann selbstverständlich, daß das extrakorporale Perfusionssystem unter sterilen Bedingungen arbeiten muß. Es wird im Hinblick auf die apparative Ausgestaltung insbesondere auf die EP 0 180 720 verwiesen.

Zusammenfassend ermöglicht das erfindungsgemäße Verfahren erstmals, auf einfache und effektive Weise die beiden Hauptmediatoren der septischen Krankheitszustände aus Patientenblut in einem extrakorporalen Perfusionssystem zu entfernen. Damit wird die fatale Kaskade unterbrochen, die ohne weitere Behandlung in der Mehrzahl der Fälle einen lethalen Ausgang für den Patienten nach sich zieht.

Die folgenden Beispiele sollen die Erfindung weiter erläutern.

### Beispiel 1

### Elimination des TNFα und eines bakteriellen Lipopolysaccharids aus Humanvollblut unter Verwendung des HELP-Aphereseverfahrens

2100 ml von gesunden Spendern frisch gewonnenes, heparinisiertes (3 IU Heparin pro ml) Humanvollblut (Hämatokrit 44 %) wurden mit 360 pg/ml an humanem, rekombinantem TNFα (Fa. Serva, Heidelberg) und mit 3,40 EU/ml (242 pg/ml) an bakteriellem Lipopolysaccharid (E.coli 055:B5 Endotoxin, Fa. BioWhittaker, Walkersville, USA) versetzt.

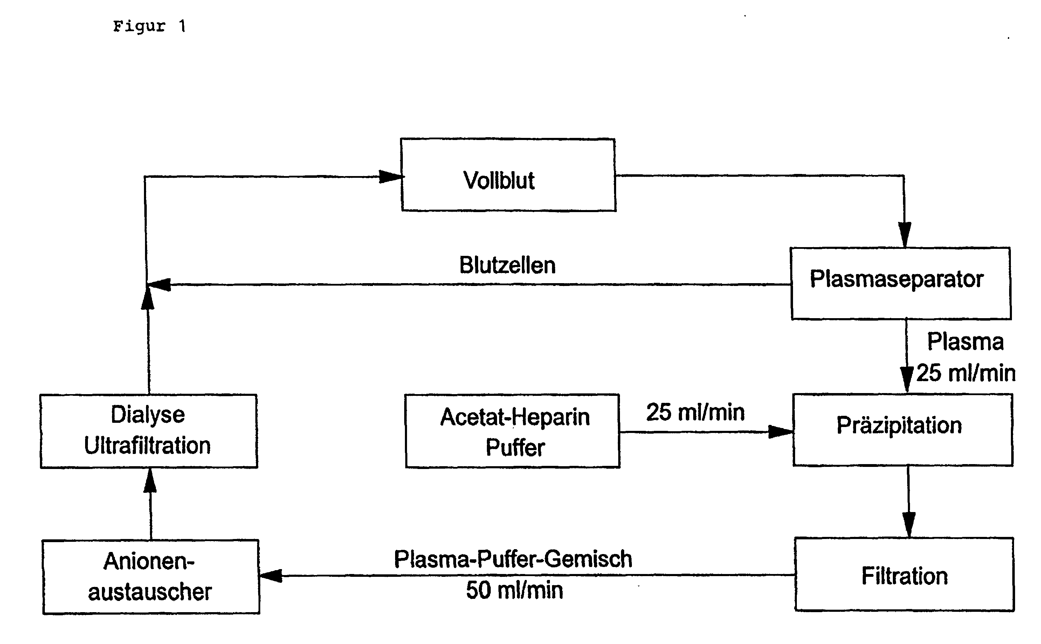

Unter Verwendung der Plasmat-Secura-Apheresemaschine der Fa. Braun (Melsungen) und deren sterilen Originaleinmalmaterials wurde das Vollblut unter rezirkulierenden Bedingungen (Figur 1) einer konventionellen HELP-Behandlung unterworfen.

Hierbei wird das Vollblut mit 70 ml/min über einen Hohlfaserplasmaseparator (Haemoselect, 0,2 m² Oberfläche, Fa. Braun, Melsungen) gepumpt und in eine Blutzell- und Plasmafraktion aufgetrennt. Das gewonnene Plasma wird in eine Mischkammer mit einem Volumenstrom von 25 ml/min geleitet und mit einem Heparin-haltigen (100 IU/ml) 0,2 M Acetatpuffer (pH 4,85) im Verhältnis 1:1 versetzt. Der bei dem resultierenden pH-Wert von 5,12 erzeugte Niederschlag besteht bekannterweise aus Heparin-Addukten der Low-Density-Lipoproteine und des Fibrinogens sowie - erfindungsgemäß - aus einem Heparin-TNFα-Addukt.

Der Niederschlag wird in einer Filterkerze (Polycarbonat-Präzipitatfilter, 1,7 m² Oberfläche, Porengröße 0,4 µm, Fa. Braun, Melsungen) quantitativ abfiltriert. Das geklärte Plasma-Puffer-Gemisch durchströmt anschließend mit einer Flußrate von 50 ml/min einen Anionenaustauscher (DEAE-Cellulose, Heparin-Adsorber, Fa. Braun, Melsungen). Dieser Verfahrensschritt führt in bekannter Weise zur Adsorption und Elimination des überschüssigen Fällungsreagenz Heparin und erfindungsgemäß zur simultanen Adsorption und Elimination bakterieller Lipopolysaccharide (Endotoxine).

Das Plasma-Puffer-Gemisch wird anschließend bei einem Volumenstrom von 50 ml/min einer Bicarbonat-Dialyse und Ultrafiltration (1,2 m² Oberfläche, Fa. Braun, Melsungen) unterworfen, wodurch die physiologischen pH-, Elektrolyt- und Volumenverhältnisse wiederhergestellt werden. Das behandelte Plasma wird abschließend bei einem Volumenstrom von 25 ml/min mit der Blutzellen-Fraktion vereinigt und in den Vollblutbeutel eingeleitet.

Nach der rezirkulierenden Perfusion von 1200 ml Plasma, dies entspricht 1 Plasmavolumen in bezug auf die vorgelegte Vollblutkonserve, wurden im behandelten Vollblut nach Korrektur der Verdünnung über den Hämatokritwert eine TNFα-Konzentration von 190 pg/ml und eine Lipopolysaccharid (Endotoxin)-Konzentration von 1,36 EU/ml (97 pg/ml) ermittelt. Die quantitative Bestimmung des TNFα erfolgte mit dem Enzym-Immunoassay TNFα-EAISA der FA. Medgenix, Ratingen. Das Lipopolysaccharid wurde mit dem chromogenen, kinetischen Limulus Amoebocyten-Lysat (LAL)-Test der Fa. Chromogenix AB, Mölndal, Schweden quantifiziert.

### Beispiel 2

### Elimination von bakteriellem Lipopolysaccharid und Heparin aus Humanplasma unter dem HELP-Verfahren analogen pH- und Puffer-Bedingungen und unter Verwendung eines makroporösen Anionenaustauschers

500 ml Humanplasma (Human-Frischplasma CPD) wurden mit 11,6 EU/ml (0,82 ng/ml) an bakteriellem Lipopolysaccharid (E.coli 055: B5 Endotoxin, Fa. BioWhittaker, Walkersville, USA) versetzt und entsprechend dem HELP-Verfahren im Verhältnis 1:1 mit einem Heparin-haltigen (100 IU/ml) 0,2 M Acetatpuffer (pH 4,85) gemischt. Der bei dem resultierenden pH-Wert von 5,12 erzeugte Niederschlag aus Heparin-Addukten der Low-Density-Lipoproteine und des Fibrinogens wurde bei 3000 g innerhalb von 10 min abzentrifugiert.

Der Überstand (960 ml Plasma/Puffer-Gemisch, pH 5,12) wurde anschließend mit einer Flußrate von 50 ml/min über eine mit dem Anionenaustauscher Lewatit® OC 1070 (Fa. Bayer AG, Leverkusen) gefüllte Kartusche (Füllvolumen: 380 ml), die mit 2000 ml einer pyrogenfreien 0,9 % Kochsalzlösung vorkonditioniert war, gepumpt.

Die quantitative Bestimmung des Lipopolysaccharids und des Heparins im perfundierten Eluat ergab, daß über 99 % des Lipopolysaccharids und über 98 % des Heparins aus dem Humanplasma durch Bindung an den Anionenaustauscher eliminiert wurden.

### Beispiel 3

### Adsorption von Plasmaproteinen unter dem HELP-Verfahren analogen pH- und Puffer-Bedingungen an einen Lewatit®-Anionenaustauscher

Entsprechend dem HELP-Verfahren wurden 500 ml Humanplasma (Human-Frischplasma CPD) mit einem Heparin-haltigen (100 IU/ml) 0,2 M Acetat-Puffer (pH 4,85) im Verhältnis 1:1 versetzt. Der bei dem resultierenden pH-Wert von 5,12 erzeugte Niederschlag aus Heparin-Addukten der Low-Density-Lipoproteine und des Fibrinogens wurde bei 3000 g innerhalb von 10 min abzentrifugiert.

Der Überstand (960 ml Plasma/Puffer-Gemisch, pH 5,12) wurde anschließend mit einer Flußrate von 50 ml/min über eine mit dem Anionenaustauscher Lewatit® OC 1070 (Fa. Bayer AG, Leverkusen) gefüllte Kartusche (Füllvolumen: 380 ml), die mit 2000 ml einer 0,9 % Kochsalz-Lösung vorkonditioniert war, gepumpt. Das im Totvolumen der Kartusche verbleibende Plasma/Puffer-Gemisch wurde unter Verwendung eines 0,1M Acetatpuffers (pH 5,12, 1000 ml, Flußrate: 50 ml/min) entfernt.

Abschließend erfolgte die Desorption der bei pH 5,12 an den Anionenaustauscher gebundenen Plasmaproteine unter Umkehr der Flußrichtung und unter rezirkulierenden Bedingungen (30 min) durch eine 2M Kochsalz-Lösung (300 ml) bei einer Flußrate von 30 ml/min.

Die quantitative Bestimmung der verschiedenen - in Tabelle 1 aufgeführten - Plasmaproteine ergab, daß nur 1,77 g an Gesamteiweiß, d.h. nur 6,1 % des Gehalts des unbehaltenden Plasma/Puffer-Gemisches, adsorptiv gebunden wurde. Von den untersuchten Proteinen wurden nur Präalbumin, Coeruloplasmin, Retinol-bindendes Protein und α₁-Glykoprotein in signifikantem Umfang eliminiert. Es ist jedoch bekannt, daß diese Plasmaproteine sehr rasch durch körpereigene Synthese substituiert werden und eine zeitlich begrenzte Reduktion keine unerwünschten physiologischen Reaktionen hervorruft.

**Tabelle 1**

| Adsorption von Plasmaproteinen unter dem HELP-Verfahren analogen pH- und Puffer-Bedingungen an einem Lewatit®-Anionen-austauscher | | |
|---|---|---|
| | [mg]^{a} | [%]^{b} |
| Gesamtprotein | 1770 | 6,1 |
| Albumin | 1050 | 6,7 |
| Präalbumin | 76 | 53,1 |
| IgA | 22 | 3,1 |
| IgG | 32 | 0,8 |
| IgM | 21 | 3,9 |
| β₂-Mikroglobulin | 0,002 | 0,8 |
| α₂-Makroglobulin | 4 | 0,7 |
| Coeruloplasmin | 36 | 33,9 |
| Haptoglobin | 9 | 2,5 |
| Haemopexin | 4 | 1,1 |
| Retinol-bindendes Protein | 10 | 55,5 |
| Ferritin | 0,002 | 1,5 |
| Transferrin | 15 | 1,2 |
| α₁-Glykoprotein | 190 | 63,3 |
| α₁-Antitrypsin | 63 | 6,7 |

| | | |
|---|---|---|
| ^{a} Adsorbierte Menge bezogen auf 1000 ml Perfusat (Humanplasma/0,2 M Acetatpuffer, pH 4,85; 1:1) | | |
| ^{b} Prozentualer Anteil gegenüber Ausgangswert | | |

## Patentansprüche

1. Verfahren zur Entfernung von Tumornekrosefaktor α (TNFα) oder/und bakteriellen Lipopolysacchariden (LPS) aus einer wäßrigen Flüssigkeit, insbesondere Blut, Blutplasma oder Serum, in einem extrakorporalen Perfusionssystem,
**dadurch gekennzeichnet,**
**daß** man nach gegebenenfalls nötiger Entfernung von korpuskulären Blutbestandteilen
(a) den pH-Wert der Körperflüssigkeit auf pH < 6 einstellt,
(b) ein Fällungsreagenz in Form eines Polyanions zugibt,
(c) gefällte Substanzen abfiltriert oder/und abzentrifugiert,
(d) die resultierende Flüssigkeit über einen Anionenaustauscher leitet.

2. Verfahren nach Anspruch 1,
**dadurch gekennzeichnet,**
**daß** man in Schritt (a) den pH-Wert der Flüssigkeit auf 4,0 bis 5,8 und insbesondere 5,05 bis 5,25 einstellt.

3. Verfahren nach Anspruch 1 oder 2,
**dadurch gekennzeichnet,**
**daß** man in Schritt (a) den pH-Wert mittels eines Puffers einstellt.

4. Verfahren nach Anspruch 3,
**dadurch gekennzeichnet,**
**daß** man einen Citratpuffer, einen Lactatpuffer, einen Acetatpuffer oder Gemische hiervon verwendet.

5. Verfahren nach einem der vorhergehenden Ansprüche,
**dadurch gekennzeichnet,**
**daß** eine Verdünnung der Körperflüssigkeit mit Pufferlösung im Verhältnis von 1:5 bis 5:1 erfolgt.

6. Verfahren nach einem der vorhergehenden Ansprüche,
**dadurch gekennzeichnet,**
**daß** man in Schritt (b) als Polyanion Heparin, hydrolysiertes Heparin, Heparinderivate oder -fragmente, sulfatiertes Glycosaminoglycan oder sulfatierte Polysaccharide oder Mischungen hiervon verwendet.

7. Verfahren nach Anspruch 6,
**dadurch gekennzeichnet,**
**daß** man als Polyanion Heparin verwendet.

8. Verfahren nach einem der vorhergehenden Ansprüche,
**dadurch gekennzeichnet,**
**daß** man in Schritt (b) das Polyanion in einer Menge von 0,001 bis 10 mg/ml bzw. 10 bis 400 IU/ml im Falle von Heparin oder seinen Derivaten, bezogen auf die Menge der Körperflüssigkeit einsetzt.

9. Verfahren nach einem der vorhergehenden Ansprüche,
**dadurch gekennzeichnet,**
**daß** man in Schritt (c) die gefällten Substanzen über einen Filter mit einer mittleren Porengröße von 0,01 bis 1,0 µm abfiltriert.

10. Verfahren nach Anspruch 9,
**dadurch gekennzeichnet,**
**daß** man eine Filterkerze verwendet.

11. Verfahren nach einem der vorhergehenden Ansprüche,
**dadurch gekennzeichnet,**
**daß** man in Schritt (c) die gefällten Substanzen mit Hilfe einer Durchflußzentrifuge abzentrifugiert.

12. Verfahren nach einem der vorhergehenden Ansprüche,
**dadurch gekennzeichnet,**
**daß** man Anionenaustauscher mit Grundträgermaterialien aus porösem Glas oder/und mit organischen Polymerisaten oder Copolymerisaten beschichtetem Kieselgel, quervernetzten Kohlehydraten oder/und organischen Polymerisaten oder Copolymerisaten verwendet.

13. Verfahren nach einem der vorhergehenden Ansprüche,
**dadurch gekennzeichnet,**
**daß** man als Anionenaustauscher ein Material verwendet, das als funktionelle Gruppen Kationen oder natürliche, synthetische oder halbsynthetische Polykationenketten enthält, wobei Polykationenketten in linearer oder verzweigter Form vorliegen können.

14. Verfahren nach Anspruch 12,
**dadurch gekennzeichnet,**
**daß** man als Kationen bzw. Polykationen tertiäre oder/und quartäre Amine verwendet.

15. Verfahren nach einem der vorhergehenden Ansprüche,
**dadurch gekennzeichnet,**
**daß** man als Anionenaustauscher quervernetzte oder/und mikrogranuläre Dialkylaminoalkyl-, Dialkylaminoaryl-, Trialkylammoniumalkyl- oder Trialkylammoniumaryl-Cellulosen oder/und Dialkylaminoalkyl-, Dialkylaminoaryl-, Trialkylammoniumalkyl- oder Trialkylammoniumaryl-modifizierte organische Polymere oder Copolymere verwendet.

16. Verfahren nach einem der vorhergehenden Ansprüche,
**dadurch gekennzeichnet,**
**daß** man in einem weiteren Schritt
(e) durch Ultrafiltration den ursprünglichen Wassergehalt der Flüssigkeit wieder herstellt oder/und durch Dialyse oder/und durch Zugabe eines geeigneten Puffers, wie beispielsweise Bicarbonat-Puffer, den physiologischen pH-Wert regeneriert.

17. Verfahren nach einem der vorhergehenden Ansprüche,
**dadurch gekennzeichnet,**
**daß** man die Dialyse in Schritt (e) gegen einen Bicarbonatpuffer durchführt.

## Claims

1. Process for the removal of tumour necrosis factor α (TNFα) or/and bacterial lipopolysaccharides (LPS) from an aqueous liquid in particular blood, blood plasma or serum in an extracorporeal perfusion system,
wherein
after removing corpuscular blood components if necessary,
(a) the pH value of the body fluid is adjusted to pH < 6,
(b) a precipitation reagent in the form of a polyanion is added
(c) precipitated substances are removed by filtration or/and centrifugation and
(d) the resulting liquid is passed over an anion exchanger.

2. Process as claimed in claim 1,
**wherein**
in step (a) the pH value of the liquid is adjusted to 4.0 to 5.8 and in particular 5.05 to 5.25.

3. Process as claimed in claim 1 or 2,
**wherein**
in step (a) the pH value is adjusted by means of a buffer.

4. Process as claimed in claim 3,
**wherein**
a citrate buffer, a lactate buffer, an acetate buffer or mixtures thereof are used.

5. Process as claimed in one of the previous claims,
**wherein**
the body liquid is diluted with buffer solution in a ratio of 1:5 to 5:1.

6. Process as claimed in one of the previous claims,
**wherein**
heparin, hydrolysed heparin, heparin derivatives or fragments, sulfated glycosaminoglycan or sulfated polysaccharides or mixtures thereof are used as the polyanion in step (b).

7. Process as claimed in claim 6,
**wherein**
heparin is used as the polyanion.

8. Process as claimed in one of the previous claims,
**wherein**
in step (b) the polyanion is used in an amount of 0.001 to 10 mg/ml or 10 to 400 IU/ml in the case of heparin or derivatives thereof relative to the amount of body fluid.

9. Process as claimed in one of the previous claims,
**wherein**
in step (c) the precipitated substances are removed by filtration over a filter with an average pore size of 0.01 to 1.0 µm.

10. Process as claimed in claim 9,
**wherein**
a candle filter is used.

11. Process as claimed in one of the previous claims,
**wherein**
in step (c) the precipitated substances are removed by centrifugation with the aid of a flow-through centrifuge.

12. Process as claimed in one of the previous claims,
**wherein**
anion exchangers with base support materials made of porous glass or/and silica gel coated with organic polymers or copolymers, cross-linked carbohydrates or/and organic polymers or copolymers are used.

13. Process as claimed in one of the previous claims,
**wherein**
a material is used as the anion exchanger which contains cations or natural, synthetic or semisynthetic polycation chains as functional groups in which polycation chains can be present in a linear or branched form.

14. Process as claimed in claim 12,
**wherein**
tertiary or/and quarternary amines are used as cations or polycations.

15. Process as claimed in one of the previous claims,
**wherein**
cross-linked or/and microgranular dialkylaminoalkyl-, dialkylaminoaryl-, trialkylammoniumalkyl- or trialkylammoniumaryl-celluloses or/and dialkylaminoalkyl-, dialkylaminoaryl-, trialkylammoniumalkyl- or trialkylammoniumaryl-modified organic polymers or copolymers are used as the anion exchanger.

16. Process as claimed in one of the previous claims,
**wherein**
in a further step
(e) the original water content of the fluid is restored by ultrafiltration or/and the physiological pH value is regenerated by dialysis or/and by addition of a suitable buffer such as for example bicarbonate buffer.

17. Process as claimed in one of the previous claims,
**wherein**
the dialysis in step (e) is carried out against a bicarbonate buffer.

## Revendications

1. Procédé pour éliminer le facteur de nécrose tumorale α (TNF α) et/ou les lipopolysaccharides (LPS) bactériennes d'un liquide aqueux, en particulier du sang, du plasma sanguin ou du sérum, dans un système de perfusion extracorporel, **caractérisé en ce que**, après avoir éventuellement éliminé des composants sanguins corpusculaires,
(a) l'on établit la valeur du pH du liquide corporel à pH < 6,
(b) l'on ajoute un réactif de précipitation sous forme d'un polyanion,
(c) l'on sépare par filtration et/ou par centrifugation les substances précipitées,
(d) l'on conduit le liquide qui en résulte sur un échangeur d'anions.

2. Procédé selon la revendication 1 **caractérisé en ce que** l'on établit à l'étape (a) la valeur du pH du liquide à une valeur de 4,0 à 5,8 et en particulier de 5,05 à 5,25.

3. Procédé selon la revendication 1 ou 2 **caractérisé en ce que** l'on établit à l'étape (a) la valeur du pH à l'aide d'un tampon.

4. Procédé selon la revendication 3 **caractérisé en ce que** l'on utilise un tampon citrate, un tampon lactate, un tampon acétate ou des mélanges de ceux-ci.

5. Procédé selon l'une des revendications précédentes **caractérisé en ce qu'**une dilution du liquide corporel a lieu avec une solution de tampon dans la proportion de 1:5 à 5:1.

6. Procédé selon l'une des revendications précédentes **caractérisé en ce que** l'on utilise à l'étape (b) en tant que polyanion l'héparine, l'héparine hydrolysée, des dérivés ou des fragments d'héparine, le glycosaminoglycane sulfaté ou des polyscaccharides sulfatés ou des mélanges de ceux-ci.

7. Procédé selon la revendication 6 **caractérisé en ce que** l'on utilise en tant que polyanion l'héparine.

8. Procédé selon l'une des revendications précédentes **caractérisé en ce que** l'on utilise à l'étape (b) le polyanion en une quantité de 0,001 à 10 mg/ml ou de 10 à 400 IU/ml dans le cas de l'héparine ou de ses dérivés par rapport à la quantité de liquide corporel.

9. Procédé selon l'une des revendications précédentes **caractérisé en ce que** l'on sépare par filtration à l'étape (c) les substances précipitées au moyen d'un filtre avec une grosseur de pore moyenne de 0,01 à 1,0 µm.

10. Procédé selon la revendication 9 **caractérisé en ce que** l'on utilise une bougie filtrante.

11. Procédé selon l'une des revendications précédentes **caractérisé en ce que** l'on sépare par centrifugation à l'étape (c) les substances précipitées à l'aide d'une centrifugeuse à écoulement.

12. Procédé selon l'une des revendications précédentes **caractérisé en ce que** l'on utilise un échangeur d'anions comportant des matériaux de support en verre poreux et/ou du gel de silice revêtu de polymérisats ou de copolymérisats organiques, des hydrates de carbone à réticulation transversale et/ou des polymérisats ou des copolymérisats organiques.

13. Procédé selon l'une des revendications précédentes **caractérisé en ce que** l'on utilise en tant qu'échangeur d'anions un matériau qui contient des cations ou des chaînes de polycatious naturels, synthétiques ou semi-synthétiques en tant que groupes fonctionnels, les chaînes de polycations pouvant se présenter sous forme linéaire ou ramifiée.

14. Procédé selon la revendication 12 **caractérisé en ce que** l'on utilise des amines tertiaires et/ou quaternaires en tant que cations ou que polycations.

15. Procédé selon l'une des revendications précédentes **caractérisé en ce que** l'on utilise en tant qu'échangeur d'anions des celluloses de dialkylaminoalkyle, de dialkylaminoaryle, de trialkylammoniumalkyle ou de trialkylammoniumaryle à réticulation transversale et/ou microgranulaires et/ou des polymères ou des copolymères organiques modifiés par dialkylaminoalkyle, par dialkylaminoaryle, par trialkylammoniumalkyle ou par trialkylammoniumaryle.

16. Procédé selon l'une des revendications précédentes **caractérisé en ce qu'**au cours d'une étape supplémentaire
(e) l'on restitue par ultrafiltration la teneur en eau originelle du liquide et/ou l'on régénère la valeur du pH physiologique par dialyse et/ou par ajout d'un tampon adapté, comme par exemple le tampon bicarbonate.

17. Procédé selon l'une des revendications précédentes **caractérisé en ce que** la dialyse à l'étape (e) est réalisée contre un tampon bicarbonate.
